# EUROPEAN PATENT APPLICATION

(11) **EP 4 810 615 A1**
(43) Date of publication of application: **23.09.2026**
(21) Application number: 25895236.5
(22) Date of filing: 12.08.2025
(51) Int. Cl.: C12N 15/77, C12N 9/00, C12N 15/52, C12P 13/10

(54) **MICROORGANISM, AND METHOD FOR PRODUCING L-ARGININE USING SAME**

(30) Priority: 21.11.2024 KR 20240167778
(71) Applicant: CJ Cheiljedang Corporation, Seoul 04560 (KR)
(72) Inventor: KIM, Ju Eun, Seoul 04560 (KR); KIM, Ye-Eun, Seoul 04560 (KR); LEE, Zeewon, Seoul 04560 (KR)
(74) Representative: Meissner Bolte Partnerschaft mbB
(86) International application number: PCT/KR2025/012160
(87) International publication number: WO 2026/111104

(57) **Abstract**

The present disclosure relates to an L-arginine-producing microorganism in which the activity of a regulator of nitrate reductase operon expression is attenuated and the activity of glutamine synthetase is enhanced, and to a method for producing L-arginine using same, wherein the L-arginine-producing microorganism, in which the activity of the regulator of nitrate reductase operon expression is attenuated and the activity of glutamine synthetase is enhanced, has been confirmed to exhibit increased L-arginine production capability compared to a parent strain, and thus can be widely used for L-arginine production.

## Description

### [TECHNICAL FIELD]

### Cross-reference to related application(s)

The present disclosure claims the benefit of priority based on Korean Patent Application No. 10-2024-0167778 filed on November 21, 2024, the entire contents of which are incorporated herein by reference as part of the present disclosure.

The present disclosure relates to an L-arginine-producing microorganism in which aerobic repressor of nitrate reductase R activity is weakened and glutamine synthetase activity is enhanced, and a method for producing L-arginine using the same.

### [BACKGROUND ART]

A microorganism of the genus *Corynebacterium* is a Gram-positive microorganism that is widely used for the production of L-amino acids.

Various studies are being conducted to develop high-efficiency production microorganisms for the production of L-amino acids and other useful substances. Specifically, for the production of L-arginine, a target substance-specific approach, such as increasing the expression of a gene encoding an enzyme mainly involved in L-arginine biosynthesis or removing a gene unnecessary for the biosynthesis of L-arginine in a strain of the genus *Corynebacterium,* has been mainly used (US Patent No. 9644009 B2).

However, with the increasing demand for L-arginine, there remains a need for a method that may efficiently produce L-arginine with high efficiency.

### [DETAILED DESCRIPTION OF THE INVENTION]

### [TECHNICAL PROBLEM]

One embodiment of the present disclosure provides a microorganism of the genus *Corynebacterium* in which an activity of an aerobic repressor of nitrate reductase R is weakened and an activity of a glutamine synthetase is enhanced.

Another embodiment of the present disclosure provides a method for producing L-arginine, comprising culturing a microorganism of the genus *Corynebacterium* in which the activity of an aerobic repressor of nitrate reductase R is weakened and the activity of a glutamine synthetase is enhanced in a medium; and recovering L-arginine from the cultured microorganism, the medium, or both.

Another embodiment of the present disclosure provides a composition for producing L-arginine, comprising a microorganism of the genus *Corynebacterium* in which an activity of an aerobic repressor of nitrate reductase R is weakened and an activity of a glutamine synthetase is enhanced.

Another embodiment of the present disclosure provides a use of a microorganism of the genus *Corynebacterium,* in which the activity of an aerobic repressor of nitrate reductase R is weakened and the activity of a glutamine synthetase is enhanced, for producing L-arginine.

### [TECHNICAL SOLUTION]

This will be described in detail as follows. Meanwhile, each description and embodiment disclosed in the present disclosure may also be applied to each other description and embodiment. That is, all combinations of various elements disclosed in the present disclosure fall within the scope of the present disclosure. In addition, the scope of the present disclosure may not be considered to be limited by the specific descriptions described below.

The present disclosure provides a microorganism having enhanced L-arginine production ability and a method for producing L-arginine using the same.

One embodiment of the present disclosure provides a microorganism of the genus *Corynebacterium* in which aerobic repressor of nitrate reductase R activity is weakened and glutamine synthetase activity is enhanced.

In the present disclosure, the term "Aerobic repressor of nitrate reductase R (a regulator of the expression level of a nitrate reductase operon)" refers to a transcriptional regulator that suppresses the expression of the nitrate reductase R operon narKGHJI. The aerobic repressor of nitrate reductase R of the present disclosure may be used interchangeably with the ArnR protein, and in the present disclosure, the sequence of the aerobic repressor of nitrate reductase R may be obtained from NCBI's GenBank, a known database. More specifically, the aerobic repressor of nitrate reductase R may have and/or comprise the amino acid sequence of SEQ ID NO: 1, or may consist essentially of or consist of the amino acid sequence.

For example, the protein consisting of the amino acid sequence of SEQ ID NO: 1 may refer to a protein intrinsically present in a microorganism of the genus *Corynebacterium* encoded by the known NCgl1138 gene, but is not limited thereto. Specifically, the protein consisting of the amino acid sequence of SEQ ID NO: 1 may refer to an aerobic repressor of nitrate reductase R derived from *Corynebacterium glutamicum* ATCC13869, which is encoded by the NCgl1138 gene, but is not limited thereto.

In addition, the aerobic repressor of nitrate reductase R of the present disclosure may comprise the amino acid sequence of SEQ ID NO: 1 as well as an amino acid sequence having at least 60%, 70%, 75%, 80%, 85%, 90%, 91%, 92%, 93%, 94%, 95%, 96%, 97%, 98%, 99%, 99.5%, or 99.9% sequence identity or homology to the amino acid sequence of SEQ ID NO: 1, as well as the amino acid sequence of SEQ ID NO: 1 itself. Furthermore, an amino acid sequence that has such sequence identity or homology and has the same or equivalent biological activity as the aerobic repressor of nitrate reductase R of the present disclosure may also be included within the scope of the present disclosure, even if some sequences are deleted, modified, substituted, conservatively substituted, or added. For example, it may include an amino acid sequence having sequence addition or deletion, a naturally occurring mutation, a silent mutation, or a conservative substitution that does not alter the activity of the aerobic repressor of nitrate reductase R at the N-terminus, C-terminus, and/or within the amino acid sequence.

In the present disclosure, the term "glutamine synthetase" may refer to an enzyme (EC: 6.3.1.2) that mediates an ATP-dependent biosynthetic reaction of glutamine from glutamate and ammonia. In the present disclosure, the term "glutamine synthetase" may be used interchangeably with "type I glutamate-ammonia ligase", "glutamine synthetase", and "GInA protein".

In one embodiment, the glutamine synthetase may be derived from a microorganism of the genus *Corynebacterium* (e.g., *Corynebacterium glutamicum*), a microorganism of the genus Aureibacillus (e.g., Aureibacillus halotolerans), or a microorganism of the genus Bacillus (e.g., Bacillus subtilis), but is not limited thereto.

In the present disclosure, the sequence of the glutamine synthetase may be obtained from the known database, NCBI GenBank (e.g., the glutamine synthetase derived from *Corynebacterium glutamicum* has the NCBI accession number "NCgl2133", the glutamine synthetase derived from Aureibacillus halotolerans has the NCBI Reference number "WP_133580410.1", and the glutamine synthetase derived from Bacillus subtilis has the NCBI Reference number "WP_003231737.1"). More specifically, the glutamine synthetase may have and/or comprise the amino acid sequence of SEQ ID NO: 3, the amino acid sequence of SEQ ID NO: 33, or the amino acid sequence of SEQ ID NO: 35, or may consist essentially of or consist of the amino acid sequence.

In addition, the glutamine synthetase of the present disclosure may comprise the amino acid sequence of SEQ ID NO: 3, SEQ ID NO: 33, or SEQ ID NO: 35, as well as an amino acid sequence having at least 60%, 70%, 75%, 80%, 85%, 90%, 91%, 92%, 93%, 94%, 95%, 96%, 97%, 98%, 99%, 99.5%, or 99.9% sequence identity or homology to the amino acid sequence of SEQ ID NO: 3, SEQ ID NO: 33, or SEQ ID NO: 35. Furthermore, any amino acid sequence having such sequence identity or homology and exhibiting the same or corresponding biological activity as the glutamine synthetase of the present disclosure, including those with deletions, modifications, substitutions, conservative substitutions, or additions of some sequences, may also be included within the scope of the present disclosure. For example, this may be a case where there is sequence addition or deletion, a naturally occurring mutation, a silent mutation, or a conservative substitution that does not alter glutamine synthetase activity at the N-terminus, C-terminus, and/or within the amino acid sequence.

The "conservative substitution" refers to substituting one amino acid with another amino acid having similar structural and/or chemical properties. Such amino acid substitutions may generally occur based on similarities in the polarity, charge, solubility, hydrophobicity, hydrophilicity and/or amphipathic nature of the residues. Typically, conservative substitutions may have little or no effect on the activity of a protein or polypeptide.

In the present disclosure, 'homology' or 'identity' refers to the degree of similarity between two given amino acid sequences or base sequences and may be expressed as a percentage. The terms homology and identity may often be used interchangeably.

Sequence homology or identity of a conserved polynucleotide or polypeptide is determined by standard alignment algorithms, and default gap penalties established by the program used may be used together. Substantially, sequences that are homologous or identical may generally hybridize to the entire sequence or a portion thereof under medium or high stringent conditions. It is self-evident that hybridization also includes hybridization with a polynucleotide containing a normal codon or a codon considering codon degeneracy in the polynucleotide.

Whether any two polynucleotide or polypeptide sequences have homology or identity may be determined, for example, using a known computer algorithm such as the "FASTA" program using default parameters as in Pearson et al (1988) [Proc. Natl. Acad. Sci. USA 85]: 2444. Alternatively, it may be determined using the Needleman-Wunsch algorithm (Needleman and Wunsch, 1970, J. Mol. Biol. 48: 443-453), as performed in the Needleman program of the EMBOSS package (EMBOSS: The European Molecular Biology Open Software Suite, Rice et al., 2000, Trends Genet. 16: 276-277) (version 5.0.0 or later), including the GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ET AL.] (1988) SIAM J Applied Math 48: 1073). For example, homology or identity may be determined using BLAST or ClustalW of the National Center for Biotechnology Information.

Homology or identity of a polynucleotide or polypeptide may be determined by comparing sequence information using a GAP computer program, such as, for example, Needleman et al. (1970), J Mol Biol. 48:443, as known in, for example, Smith and Waterman, Adv. Appl. Math (1981) 2:482. In summary, the GAP program may be defined as the value obtained by dividing the number of similar arranged symbols (i.e., nucleotides or amino acids) by the total number of symbols in the shorter of the two sequences. Default parameters for the GAP program may include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix), as disclosed by Schwartz and Dayhoff, eds., Atlas Of Protein Sequence And Structure, National Biomedical Research Foundation, pp. 353-358 (1979); (2) a penalty of 3.0 for each gap and an additional penalty of 0.10 for each symbol in each gap (or gap opening penalty 10, gap extension penalty 0.5); and (3) no penalty for terminal gaps.

In the present disclosure, the term "L-arginine" refers to a conditionally essential amino acid present in all organisms, meaning an L-amino acid having a chemical formula of C₆H₁₄N₄O₂.

In the present disclosure, the term "microorganism (or strain)" encompasses both wild-type microorganisms and microorganisms that have undergone natural or artificial genetic modification, and refers to a microorganism in which a specific mechanism is enhanced or weakened due to reasons such as the insertion of a foreign gene, or the enhancement or weakening of the activity of an endogenous gene, and may be a microorganism that comprises a genetic modification for the production of a desired polypeptide, protein, or product.

In the present disclosure, the term "enhancement" of polypeptide activity means that the activity of a polypeptide is increased compared to its intrinsic activity. The term enhancement may be used interchangeably with terms such as activation, up-regulation, overexpression, and increase. Here, activation, enhancement, up-regulation, overexpression, and increase may all include exhibiting an activity that was not originally possessed, or exhibiting an activity that is improved compared to an intrinsic activity or an activity before modification. The "intrinsic activity" refers to the activity of a specific polypeptide originally possessed by a parent strain or an unmodified microorganism before a trait is changed due to genetic variation caused by natural or artificial factors. This term may be used interchangeably with "activity before modification". That the activity of a polypeptide is "enhanced," "up-regulated," "overexpressed," or "increased" compared to the intrinsic activity means that it is improved compared to the activity and/or concentration (expression level) of a specific polypeptide originally possessed by the parent strain or unmodified microorganism before the trait change.

The enhancement may be achieved through introduction of a foreign polypeptide or through enhancement of activity and/or increase in concentration (expression level) of an endogenous polypeptide. Whether the activity of the polypeptide is enhanced may be confirmed from an increase in the degree of activity of the polypeptide, the expression level, or the amount of a product due to the activity of the polypeptide.

Enhancement of the activity of the polypeptide may be achieved by applying various methods well known in the art, and is not limited as long as the activity of the target polypeptide may be enhanced compared to that of the microorganism before modification. Specifically, it may be performed using genetic engineering and/or protein engineering well known to those skilled in the art, which are routine methods of molecular biology, but is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be:
1) an increase in the intracellular copy number of a polynucleotide encoding the polypeptide;
2) replacement of a gene expression regulatory region of the gene encoding the polypeptide on a chromosome with a sequence having strong activity;
3) modification of a nucleotide sequence encoding a start codon or a 5'-UTR region of a gene transcript encoding the polypeptide;
4) modification of the amino acid sequence of the polypeptide to enhance polypeptide activity;
5) modification of the polynucleotide sequence encoding the polypeptide to enhance the activity of the polypeptide (e.g., modification of the polynucleotide sequence of the gene encoding the polypeptide to encode a polypeptide modified to enhance the activity thereof);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select an exposed site and modify or chemically modify the same; or
9) regulation of intracellular localization of the protein (polypeptide); or
10) a combination of two or more selected from the above items 1) to 9), but is not particularly limited thereto.

More specifically, the increase in the intracellular copy number of the polynucleotide encoding the polypeptide as described in item 1) may be achieved by introducing into a host cell a vector, to which the polynucleotide encoding the polypeptide is operably linked and which may replicate and function independently of the host. Alternatively, it may be achieved by introducing one copy or two or more copies of the polynucleotide encoding the polypeptide into the chromosome of the host cell. The introduction into the chromosome may be performed by introducing into the host cell a vector capable of inserting the polynucleotide into the chromosome of the host cell, but is not limited thereto.

The replacement of the gene expression regulatory region (or expression regulatory sequence) of a gene encoding the polypeptide on a chromosome with a sequence having strong activity as described in item 2) may be, for example, creating a sequence variation by deletion, insertion, non-conservative or conservative substitution, or a combination thereof to further enhance the activity of the expression regulatory region, or replacement with a sequence having stronger activity. The expression regulatory region may include, but is not particularly limited to, a promoter, an operator sequence, a sequence encoding a ribosome binding site, and a sequence regulating the termination of transcription and translation. For example, it may be replacing the original promoter with a strong promoter, but is not limited thereto.

Examples of known strong promoters include CJ1 to CJ7 promoters (US Patent No. 7662943 B2), lac promoter, trp promoter, trc promoter, tac promoter, lambda phage PR promoter, PL promoter, tet promoter, gapA promoter, SPL7 promoter, SPL13 (sm3) promoter (US Patent No. 10584338 B2), O2 promoter (US Patent No. 10273491 B2), tkt promoter, yccA promoter, lysCP1 promoter (US Patent No. 8426577 B2), and the like, but are not limited thereto.

The modification of the nucleotide sequence encoding the start codon or 5'-UTR region of the gene transcript encoding the polypeptide as described in item 3) may be, for example, substitution with a nucleotide sequence encoding another start codon having a higher polypeptide expression rate compared to the endogenous start codon, but is not limited thereto.

The modification of the amino acid sequence or polynucleotide sequence as described in items 4) and 5) above may be a sequence variation caused by deletion, insertion, non-conservative or conservative substitution, or a combination thereof in the amino acid sequence of the polypeptide or the polynucleotide sequence encoding the polypeptide to enhance the activity of the polypeptide, or a replacement with an amino acid sequence or a polynucleotide sequence improved to have stronger activity or an amino acid sequence or a polynucleotide sequence improved to have increased activity, but is not limited thereto. Specifically, the replacement may be performed by inserting the polynucleotide into the chromosome by homologous recombination, but is not limited thereto. The vector used herein may further comprise a selection marker for confirming chromosomal insertion.

The introduction of a foreign polynucleotide exhibiting the activity of the polypeptide as described in item 6) may be the introduction of a foreign polynucleotide, which encodes a polypeptide exhibiting the same or similar activity as the polypeptide, into a host cell. There is no restriction on the origin or sequence of the foreign polynucleotide as long as it exhibits the same or similar activity as the polypeptide. The method used for the introduction may be performed by a person skilled in the art appropriately selecting a known transformation method, and the activity of the polypeptide may be increased by generating the polypeptide through the expression of the introduced polynucleotide in the host cell.

The codon optimization of the polynucleotide encoding the polypeptide as described in item 7) may be codon optimization of an endogenous polynucleotide to increase transcription or translation in the host cell, or codon optimization of a foreign polynucleotide to achieve optimized transcription and translation in the host cell.

The analysis of the tertiary structure of the polypeptide to select an exposed site and modify or chemically modify it as described in item 8) may be, for example, comparing sequence information of the polypeptide to be analyzed with a database in which sequence information of known proteins is stored, determining a template protein candidate according to the degree of sequence similarity, confirming the structure based thereon, and selecting an exposed site to be modified or chemically modified to perform said modification or chemical modification.

The regulation of the intracellular localization of the protein (polypeptide) described in item 9) may be the targeting of the protein (polypeptide) to a specific intracellular organelle or a specific space within the cell. For example, it may be targeting to the periplasm or cytoplasm through the addition or removal of a leader sequence that functions to target the protein (polypeptide), but is not limited thereto.

Such enhancement of polypeptide activity may be an increase in the activity or concentration (expression level) of the corresponding polypeptide relative to the activity or concentration level of the polypeptide expressed in the wild-type or pre-modification microorganism strain, or an increase in the amount of a product produced from the polypeptide, but is not limited thereto.

In the present disclosure, the term "weakened" of a polypeptide encompasses both cases where the activity is reduced compared to the intrinsic activity and cases where there is no activity.

The term 'weakening' may be used interchangeably with terms such as 'inactivation', 'deficiency', 'down-regulation', 'decrease', 'reduction', and 'attenuation'.

The weakening may also include a case where the activity of the polypeptide itself is reduced or eliminated compared to the activity of the polypeptide originally possessed by the microorganism due to a mutation in the polynucleotide encoding the polypeptide, a case where the overall polypeptide activity level and/or concentration (expression level) in the cell is lower than that of the natural strain due to inhibition of expression of the gene encoding the polynucleotide or inhibition of translation into the polypeptide, a case where the polynucleotide is not expressed at all, and/or a case where there is no polypeptide activity even if the polynucleotide is expressed. The term "intrinsic activity" refers to the activity of a specific polypeptide originally possessed by a parent strain, a wild-type microorganism, or an unmodified microorganism before the trait is changed due to genetic variation caused by natural or artificial factors. This term may be used interchangeably with "activity before modification". The expression that the activity of a polypeptide is "inactivated, deficient, decreased, down-regulated, reduced, or weakened" compared to the intrinsic activity means that it is lower than the activity of a specific polypeptide originally possessed by the parent strain or unmodified microorganism before the trait change.

Such weakening of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the polypeptide may be:
1) deletion of all or part of a gene encoding the polypeptide;
2) modification of an expression regulatory region (or expression regulatory sequence) such that the expression of the gene encoding the polypeptide is decreased;
3) modification of the amino acid sequence constituting the polypeptide such that activity of the polypeptide is removed or weakened (e.g., deletion/substitution/addition of one or more amino acids in the amino acid sequence);
4) modification of a gene sequence encoding the polypeptide such that activity of the polypeptide is removed or weakened (e.g., deletion/substitution/addition of one or more nucleotides on the nucleic acid sequence of the gene encoding the polypeptide so as to encode a polypeptide modified such that activity of the polypeptide is removed or weakened);
5) modification of a nucleotide sequence encoding a start codon or a 5'-UTR region of a transcript of the gene encoding the polypeptide;
6) introduction of an antisense oligonucleotide (e.g., antisense RNA) that complementarily binds to a transcript of the gene encoding the polypeptide;
7) addition of a sequence complementary to a Shine-Dalgarno sequence at a front end of the Shine-Dalgarno sequence of the gene encoding the polypeptide to form a secondary structure to which ribosomes cannot attach;
8) addition of a promoter (Reverse transcription engineering, RTE) transcribed in an opposite direction to the 3' end of the open reading frame (ORF) of the gene sequence encoding the polypeptide; or
9) regulation of the intracellular localization of the protein (polypeptide); or
10) a combination of two or more selected from the above items 1) to 9), but is not particularly limited thereto.

For example, the deletion of all or part of the gene encoding the polypeptide as described in item 1) may be the removal of the entire polynucleotide encoding an endogenous target polypeptide in the chromosome, replacement with a polynucleotide in which some nucleotides are deleted, or replacement with a marker gene.

In addition, the modification of the expression regulatory region (or expression regulatory sequence) as described in item 2) may be the occurrence of a variation on the expression regulatory region (or expression regulatory sequence) by deletion, insertion, non-conservative or conservative substitution, or a combination thereof, or replacement with a sequence having weaker activity. The expression regulatory region may include, but is not limited to, a promoter, an operator sequence, a sequence encoding a ribosome binding site, and a sequence regulating the termination of transcription and translation.

In addition, the modification of the nucleotide sequence encoding the start codon or 5'-UTR region of the gene transcript encoding the polypeptide as described in item 3) may be, for example, substitution with a nucleotide sequence encoding another start codon having a lower polypeptide expression rate compared to the endogenous start codon, but is not limited thereto.

In addition, the modification of the amino acid sequence or polynucleotide sequence as described in items 4) and 5) above may be a sequence variation caused by deletion, insertion, non-conservative or conservative substitution, or a combination thereof in the amino acid sequence of the polypeptide or the polynucleotide sequence encoding the polypeptide to weaken the activity of the polypeptide, or a replacement with an amino acid sequence or a polynucleotide sequence modified to have weaker activity or an amino acid sequence or a polynucleotide sequence modified to have no activity, but is not limited thereto. For example, the expression of a gene may be inhibited or weakened by introducing a mutation into the polynucleotide sequence to form a stop codon, but is not limited thereto. The "stop codon" is a codon that acts as a signal indicating that the protein synthesis process is finished without designating an amino acid among codons on mRNA, and generally three types, UAA, UAG, and UGA, may be used as stop codons.

The introduction of the antisense oligonucleotide (e.g., antisense RNA) that complementarily binds to the transcript of the gene encoding the polypeptide as described in item 6) may be carried out by referring to, for example, the literature [Weintraub, H. et al., Antisense-RNA as a molecular tool for genetic analysis, Reviews - Trends in Genetics, Vol. 1(1) 1986].

The addition of a sequence complementary to a Shine-Dalgarno sequence upstream of the Shine-Dalgarno sequence of the gene encoding the polypeptide, to form a secondary structure to which a ribosome cannot attach, as described in item 7), may render mRNA translation impossible or reduce the rate thereof.

The addition of a promoter (Reverse transcription engineering, RTE) to the 3' end of the open reading frame (ORF) of the gene sequence encoding the polypeptide, wherein the promoter is transcribed in the opposite direction, as described in item 8), may weaken the activity by producing an antisense nucleotide complementary to the transcript of the gene encoding the polypeptide.

The regulation of the intracellular localization of the protein (polypeptide) as described in item 9) may comprise targeting the protein (polypeptide) to a specific intracellular organelle or a specific space within the cell. For example, the regulation may involve targeting to the periplasm or cytoplasm through the addition or removal of a leader sequence that functions to target the protein (polypeptide), but is not limited thereto.

Such weakening of polypeptide activity may be characterized in that the activity or concentration (expression level) of the corresponding polypeptide is reduced relative to the activity or concentration of the polypeptide expressed in the wild-type or pre-modification microorganism strain, or that the amount of a product produced from the polypeptide is reduced, but is not limited thereto.

Specifically, the L-arginine-producing microorganism of the present disclosure may be a genetically modified microorganism in which aerobic repressor of nitrate reductase R activity is weakened (e.g., removed or inactivated) and glutamine synthetase activity is enhanced, so that the L-arginine production ability (or production amount) is increased (enhanced). Specifically, the microorganism with increased L-arginine production ability (recombinant strain) may be a microorganism that has increased L-arginine production ability compared to a natural wild-type microorganism or an unmodified microorganism of the same species that has intrinsic aerobic repressor of nitrate reductase R activity (aerobic repressor of nitrate reductase R activity is not weakened) and in which glutamine synthetase activity is not enhanced, but is not limited thereto.

In the present disclosure, the term "unmodified microorganism" does not exclude strains containing mutations that may naturally occur in microorganisms, and may refer to a wild-type strain or a natural strain itself, or a strain before its characteristics are changed due to genetic variation by natural or artificial factors. For example, the unmodified microorganism may refer to a strain in which aerobic repressor of nitrate reductase R activity described in the present disclosure is not weakened or has not yet been weakened compared to the intrinsic activity, and/or in which glutamine synthetase activity is not enhanced or has not yet been enhanced compared to the intrinsic activity. The "unmodified microorganism" may be used interchangeably with "pre-modification strain", "pre-modification microorganism", "non-mutated strain", "unmodified strain", "non-mutated microorganism", or "reference microorganism", and may be referred to as a "parent microorganism or parent strain".

In one embodiment, the parent strain may be a wild-type strain, or one mutated to increase L-arginine production ability, for example, one in which protein activity involved in L-arginine biosynthesis or metabolism is regulated (increased (promoted) or decreased (inhibited)) compared to the wild-type, but is not limited thereto.

In one embodiment, the parent strain may be a microorganism naturally having L-arginine production ability, or it may be any microorganism of the genus *Corynebacterium* or the genus *Escherichia* that has acquired an L-arginine production ability through the introduction of a mutation into a parent strain that lacks or has significantly low L-arginine production ability. The microorganism of the genus *Corynebacterium* may include *Corynebacterium glutamicum, Corynebacterium stationis, Brevibacterium lactofermentum, Brevibacterium flavum, Corynebacterium thermoaminogenes, Corynebacterium efficiens,* and the like, but is not limited thereto. More specifically, the microorganism of the genus *Corynebacterium* may be *Corynebacterium glutamicum,* and the strain of the genus *Escherichia* may be *Escherichia coli.*

In one embodiment, a method of deleting all or a portion of a gene encoding a polypeptide may be used to weaken the activity of aerobic repressor of nitrate reductase R. Specifically, it may be performed by replacing a polynucleotide encoding an endogenous target protein in a chromosome with a polynucleotide in which some nucleotide sequences are deleted or with a marker gene, using a vector for chromosomal insertion in the microorganism. As an example of a method of deleting a portion or all of such a polynucleotide, a method of deleting the polynucleotide by homologous recombination may be used, but it is not limited thereto.

The gene deletion method includes a method using a genetic recombination technique. For example, it may be accomplished by introducing a polynucleotide sequence or vector containing a polynucleotide sequence homologous to a target gene into the microorganism to cause homologous recombination. In addition, the introduced polynucleotide sequence or vector may include a dominant selection marker.

In one embodiment, a method of increasing the copy number or enhancing the activity of a promoter may be used to enhance the activity of the glutamine synthetase, but it is not limited thereto.

One embodiment of the present disclosure provides a microorganism producing L-arginine, in which aerobic repressor of nitrate reductase R activity is weakened, the glutamine synthetase activity is enhanced, and in which the activities of malate:quinone oxidoreductase, malate dehydrogenase, aspartate transaminase, and NADP-specific glutamate dehydrogenase are further enhanced compared to an unmodified microorganism.

In the present disclosure, the term "malate:quinone oxidoreductase" refers to an enzyme in the TCA cycle that catalyzes the reversible conversion of malate and quinone to quinol and oxaloacetate. The malate:quinone oxidoreductase of the present disclosure may be used interchangeably with MQO protein, and the sequence of the malate:quinone oxidoreductase in the present disclosure may be obtained from NCBI's GenBank, a known database. More specifically, the malate:quinone oxidoreductase may have and/or comprise the amino acid sequence of SEQ ID NO: 54, or may consist essentially of or consist of the amino acid sequence.

For example, the protein consisting of the amino acid sequence of SEQ ID NO: 54 may refer to a protein intrinsically present in a microorganism of the genus *Corynebacterium* encoded by the known NCgl1926 gene, but it is not limited thereto. Specifically, the protein consisting of the amino acid sequence of SEQ ID NO: 54 may refer to a malate:quinone oxidoreductase derived from *Corynebacterium glutamicum* ATCC13032 encoded by the NCgl1926 gene, but is not limited thereto.

In addition, the malate:quinone oxidoreductase of the present disclosure may comprise the amino acid sequence of SEQ ID NO: 54, as well as an amino acid sequence having at least 60%, 70%, 75%, 80%, 85%, 90%, 91%, 92%, 93%, 94%, 95%, 96%, 97%, 98%, 99%, 99.5%, or 99.9% sequence identity or homology to the amino acid sequence of SEQ ID NO: 54. Furthermore, provided that an amino acid sequence has such sequence identity or homology and exhibits the same or corresponding biological activity as the malate:quinone oxidoreductase of the present disclosure, an amino acid sequence in which some sequences are deleted, modified, substituted, conservatively substituted, or added shall also be included within the scope of the present disclosure.

In the present disclosure, the term "malate dehydrogenase" refers to an enzyme in the TCA cycle that reversibly catalyzes the oxidation of malate to oxaloacetate by reducing NAD+ to NADH. The malate dehydrogenase of the present disclosure may be used interchangeably with MDH protein, and the sequence of the malate dehydrogenase in the present disclosure may be obtained from GenBank of the NCBI, a known database. More specifically, the malate dehydrogenase may have and/or comprise the amino acid sequence of SEQ ID NO: 58, or may consist essentially of or consist of the amino acid sequence.

For example, the protein consisting of the amino acid sequence of SEQ ID NO: 58 may refer to a protein intrinsically present in a microorganism of the genus *Corynebacterium* encoded by the known NCgl0631 gene, but is not limited thereto. Specifically, the protein consisting of the amino acid sequence of SEQ ID NO: 58 may mean a malate dehydrogenase derived from *Corynebacterium glutamicum* ATCC13032 encoded by the NCgl0631 gene, but is not limited thereto.

In addition, the malate dehydrogenase of the present disclosure may comprise the amino acid sequence of SEQ ID NO: 58, as well as an amino acid sequence having at least 60%, 70%, 75%, 80%, 85%, 90%, 91%, 92%, 93%, 94%, 95%, 96%, 97%, 98%, 99%, 99.5%, or 99.9% sequence identity or homology to the amino acid sequence of SEQ ID NO: 58. Furthermore, provided that an amino acid sequence has such sequence identity or homology and has the same or corresponding biological activity as the malate dehydrogenase of the present disclosure, an amino acid sequence in which some sequences are deleted, modified, substituted, conservatively substituted, or added shall also be included within the scope of the present disclosure.

In the present disclosure, the term "aspartate transaminase" refers to an enzyme that catalyzes a reaction of transferring an amino group of L-aspartic acid to 2-oxoglutarate to produce L-glutamic acid and oxaloacetic acid. The aspartate transaminase of the present disclosure may be used interchangeably with AspB protein, and the sequence of the aspartate transaminase in the present disclosure may be obtained from NCBI's GenBank, a known database. More specifically, the aspartate transaminase may have and/or comprise the amino acid sequence of SEQ ID NO: 60, or may consist essentially of or consist of the amino acid sequence.

For example, the protein consisting of the amino acid sequence of SEQ ID NO: 60 may refer to a protein intrinsically present in a microorganism of the genus *Corynebacterium* encoded by the known NCgl0237 gene, but is not limited thereto. Specifically, the protein consisting of the amino acid sequence of SEQ ID NO: 60 may mean an aspartate transaminase derived from *Corynebacterium glutamicum* ATCC13032 encoded by the NCgl0237 gene, but is not limited thereto.

In addition, the aspartate transaminase of the present disclosure may comprise the amino acid sequence of SEQ ID NO: 60, as well as an amino acid sequence having at least 60%, 70%, 75%, 80%, 85%, 90%, 91%, 92%, 93%, 94%, 95%, 96%, 97%, 98%, 99%, 99.5%, or 99.9% sequence identity or homology to the amino acid sequence of SEQ ID NO: 60. Furthermore, an amino acid sequence having such sequence identity or homology and having the same or corresponding biological activity as the aspartate transaminase of the present disclosure, even if some sequences are deleted, modified, substituted, conservatively substituted, or added, may also be included within the scope of the present disclosure.

In the present disclosure, the term "NADP-specific glutamate dehydrogenase" refers to an enzyme that catalyzes a reaction of converting glutamate to 2-oxoglutarate and ammonia in an NADP-dependent manner. The NADP-specific glutamate dehydrogenase of the present disclosure may be used interchangeably with GDH protein, and the sequence of the NADP-specific glutamate dehydrogenase of the present disclosure may be obtained from NCBI's GenBank, a known database. More specifically, the NADP-specific glutamate dehydrogenase may have and/or comprise the amino acid sequence of SEQ ID NO: 62, or may consist essentially of or consist of the amino acid sequence.

For example, the protein consisting of the amino acid sequence of SEQ ID NO: 62 may refer to a protein intrinsically present in a microorganism of the genus *Corynebacterium* encoded by the known NCgl1999 gene, but is not limited thereto. Specifically, the protein consisting of the amino acid sequence of SEQ ID NO: 62 may refer to an NADP-specific glutamate dehydrogenase derived from *Corynebacterium glutamicum* ATCC13032 encoded by the NCgl1999 gene, but is not limited thereto.

In addition, the NADP-specific glutamate dehydrogenase of the present disclosure may comprise the amino acid sequence of SEQ ID NO: 62, as well as an amino acid sequence having at least 60%, 70%, 75%, 80%, 85%, 90%, 91%, 92%, 93%, 94%, 95%, 96%, 97%, 98%, 99%, 99.5%, or 99.9% sequence identity or homology to the amino acid sequence of SEQ ID NO: 62. Furthermore, any amino acid sequence having such sequence identity or homology and having the same or corresponding biological activity as the NADP-specific glutamate dehydrogenase of the present disclosure, such as an amino acid sequence in which some sequences are deleted, modified, substituted, conservatively substituted, or added, may also be included within the scope of the present disclosure.

In one specific embodiment, it was confirmed that L-arginine production ability was increased as a result of simultaneously enhancing the activities of malate:quinone oxidoreductase, malate dehydrogenase, aspartate transaminase, and NADP-specific glutamate dehydrogenase, in addition to the weakening of aerobic repressor of nitrate reductase R activity and the enhancement of glutamine synthetase activity. Specifically, it was confirmed that the L-arginine production ability of an L-arginine-producing microorganism, into which a gene combination encoding fumarate hydratase, malate:quinone oxidoreductase, malate dehydrogenase, aspartate transaminase, and NADP-specific glutamate dehydrogenase was introduced using a plasmid, was further enhanced compared to an L-arginine-producing microorganism in which aerobic repressor of nitrate reductase R activity was weakened and glutamine synthetase activity was enhanced.

The "L-arginine", "microorganism", "weakening of activity", and "enhancement of activity" are as described above.

Another embodiment of the present disclosure provides a method for increasing L-arginine production ability of a microorganism or a method for imparting L-arginine production ability to the microorganism, comprising weakening aerobic repressor of nitrate reductase R activity in the microorganism; and enhancing glutamine synthetase activity in the microorganism.

Another embodiment of the present disclosure provides a method for producing L-arginine, comprising culturing, in a medium, a microorganism in which aerobic repressor of nitrate reductase R activity is weakened and glutamine synthetase activity is enhanced.

In the present disclosure, "culturing" means growing a microorganism of the genus *Corynebacterium,* such as a *Corynebacterium glutamicum* strain, in which aerobic repressor of nitrate reductase R activity of the present disclosure is weakened and glutamine synthetase activity is enhanced under appropriately controlled environmental conditions. The culturing process of the present disclosure may be performed according to a suitable medium and culturing conditions known in the art. Such a culturing process may be easily adjusted and used by those skilled in the art according to the selected strain. Specifically, the culturing may be batch, continuous and/or fed-batch, but is not limited thereto.

In the present disclosure, "medium" refers to a substance mixed with nutritional substances required for culturing a microorganism of the genus *Corynebacterium,* such as a *Corynebacterium glutamicum* strain, in which aerobic repressor of nitrate reductase R activity of the present disclosure is weakened and glutamine synthetase activity is enhanced, as main components; and the medium supplies nutritional substances and growth factors, including water, which are essential for survival and growth. Specifically, any medium used for culturing common microorganisms may be used as the medium and other culturing conditions for the microorganism of the present disclosure without particular limitation; however, the microorganism of the present disclosure may be cultured while adjusting temperature, pH, and the like under aerobic conditions in a common medium containing an appropriate carbon source, nitrogen source, phosphorus source, inorganic compound, amino acid, and/or vitamin.

Specifically, culture media for the microorganism of the present disclosure, such as strains of the genus *Corynebacterium,* may be found in the literature ["Manual of Methods for General Bacteriology" by the American Society for Bacteriology (Washington D.C., USA, 1981)].

In the present disclosure, the carbon source may include carbohydrates such as glucose, saccharose, lactose, fructose, sucrose, maltose, and the like; sugar alcohols such as mannitol, sorbitol, and the like; organic acids such as pyruvic acid, lactic acid, citric acid, and the like; or amino acids such as glutamic acid, methionine, lysine, and the like. In addition, natural organic nutrient sources such as starch hydrolysate, molasses, blackstrap molasses, rice bran, cassava, sugarcane residue, and corn steep liquor may be used; specifically, carbohydrates such as glucose and sterilized pre-treated molasses (i.e., molasses converted to reducing sugar) may be used; and other appropriate amounts of carbon sources may be used without limitation. These carbon sources may be used alone or in combination of two or more, but the present disclosure is not limited thereto.

The nitrogen source may include inorganic nitrogen sources such as ammonia, ammonium sulfate, ammonium chloride, ammonium acetate, ammonium phosphate, ammonium carbonate, ammonium nitrate, and the like; and organic nitrogen sources such as amino acids (e.g., glutamic acid, methionine, and glutamine), peptone, NZ-amine, meat extract, yeast extract, malt extract, corn steep liquor, casein hydrolysate, fish or decomposition products thereof, and defatted soybean cake or decomposition products thereof. These nitrogen sources may be used alone or in combination of two or more, but are not limited thereto.

The phosphorus source may include potassium phosphate monobasic, potassium phosphate dibasic, or corresponding sodium-containing salts. As inorganic compounds, sodium chloride, calcium chloride, iron chloride, magnesium sulfate, iron sulfate, manganese sulfate, calcium carbonate, and the like may be used, and in addition, amino acids, vitamins and/or appropriate precursors may be included. These components or precursors may be added to the medium batchwise or continuously. However, they are not limited thereto.

In addition, compounds such as ammonium hydroxide, potassium hydroxide, ammonia, phosphoric acid, and sulfuric acid may be added to the medium in an appropriate manner during culturing of the microorganism of the present disclosure to adjust the pH of the medium. In addition, during culture, an antifoaming agent such as a fatty acid polyglycol ester may be used to suppress foam generation. In addition, in order to maintain the aerobic state of the medium, oxygen or an oxygen-containing gas may be injected into the medium; alternatively, to maintain anaerobic and microaerobic conditions, gas injection may be omitted, or nitrogen, hydrogen, or carbon dioxide gas may be injected, but are not limited thereto.

In the culture of the present disclosure, the culture temperature may be maintained at 20 to 45°C, specifically 25 to 40°C, and culturing may be performed for about 10 to 160 hours, but is not limited thereto.

L-arginine produced by the culture of the present disclosure may be secreted into the medium or remain in the cells.

The L-arginine production method of the present disclosure may further comprise preparing the microorganism (strain) of the present disclosure, preparing a medium for culturing the microorganism, or a combination thereof (in any order), for example, prior to the culturing step.

The method for producing L-arginine of the present disclosure may further include recovering L-arginine from the medium (i.e., the medium in which culturing was performed) or the microorganism (e.g., a strain of the genus *Corynebacterium*). The recovering step may be performed after the culturing step.

The recovery may be collecting the desired L-arginine using a suitable method known in the art according to the culturing method of the microorganism of the present disclosure, for example, batch, continuous, or fed-batch cultivation methods. For example, centrifugation, filtration, treatment with a crystallization protein precipitant (salting-out method), extraction, ultrasonic disruption, ultrafiltration, dialysis, various chromatography methods such as molecular sieve chromatography (gel filtration), adsorption chromatography, ion exchange chromatography, affinity chromatography, HPLC, or a combination of these methods may be used, and the desired L-arginine may be recovered from the medium or the microorganism using a suitable method known in the art.

In addition, the L-arginine production method of the present disclosure may additionally include a purification step. The purification may be performed using a suitable method known in the art. In one embodiment, when the L-arginine production method of the present disclosure includes both a recovery step and a purification step, the recovery step and the purification step may be performed continuously or discontinuously regardless of the order, or may be performed simultaneously or integrated into one step, but are not limited thereto.

In the method for producing L-arginine of the present disclosure, the microorganism (strain), the medium, L-arginine, and the like are as described in the other examples above.

Another embodiment of the present disclosure is to provide a composition for producing L-arginine comprising the microorganism of the present disclosure, a medium in which the microorganism has been cultured, or a combination thereof.

The composition of the present disclosure may further comprise any suitable excipient commonly used in a composition for producing L-arginine, and such excipients may be, for example, a preservative, a wetting agent, a dispersing agent, a suspending agent, a buffering agent, a stabilizer, or an isotonic agent, but are not limited thereto.

In the composition of the present disclosure, the microorganism (strain), the medium, and L-arginine, and the like are as described in the other examples above.

Another embodiment provides a use of a microorganism of the genus *Corynebacterium,* in which aerobic repressor of nitrate reductase R activity is weakened and glutamine synthetase activity is enhanced, for producing L-arginine.

Another embodiment provides a use of a microorganism of the genus *Corynebacterium,* in which aerobic repressor of nitrate reductase R activity is weakened and glutamine synthetase activity is enhanced, for preparing a composition for producing L-arginine.

Another embodiment of the present disclosure provides a microorganism, method, composition, product, process, or use characterized by one or more elements disclosed in the present disclosure.

### [ADVANTAGEOUS EFFECTS]

The present disclosure relates to an L-arginine-producing microorganism in which aerobic repressor of nitrate reductase R activity is weakened and glutamine synthetase activity is enhanced, and a method for producing L-arginine using the same. It was confirmed that the L-arginine-producing microorganism in which aerobic repressor of nitrate reductase R activity is weakened and glutamine synthetase activity is enhanced has increased L-arginine production ability compared to a parent strain, and thus the microorganism may be widely utilized for L-arginine production.

### [MODE FOR INVENTION]

Hereinafter, the present disclosure will be described in more detail with reference to examples, but these are merely exemplary and are not intended to limit the scope of the present disclosure. It is obvious to those skilled in the art that the examples described below may be modified without departing from the essential spirit of the disclosure.

### Example 1. Construction of a plasmid for weakening of aerobic repressor of nitrate reductase R activity

In order to confirm the effect of weakening of the aerobic repressor of nitrate reductase R gene (hereinafter, *arnR*) of *Corynebacterium glutamicum* on L-arginine production, a vector in which the BBD29_RS06340 gene (SEQ ID NO: 2), having NCBI accession number NCgl1138 and derived from *Corynebacterium glutamicum* ATCC13869, is deleted, was constructed as follows.

Specifically, to construct the vector, PCR was performed using each of the primer pairs of SEQ ID NOs: 6 and 7 and SEQ ID NOs: 8 and 9, using the chromosome of the *Corynebacterium glutamicum* ATCC13869 strain as a template.

PfuUltra^{™} high-fidelity DNA polymerase (Stratagene) was used as a polymerase for the PCR reaction, and as PCR conditions, denaturation was performed at 95°C for 30 seconds; annealing was performed at 55°C for 30 seconds; and a polymerization reaction was performed at 72°C for 1 minute, and the denaturation, annealing, and polymerization reactions under these conditions were repeated 28 times. As a result, DNA fragments of 985 bp and 964 bp were obtained, respectively. The obtained DNA products were purified using a PCR purification kit (PCR Purification kit, QUIAGEN), and the purified amplification products were treated with the restriction enzyme Smal, and then cloned using a pDC24 vector (SEQ ID NO: 80) heat-treated at 65°C for 20 minutes and an Infusion cloning kit (Infusion Cloning Kit, TaKaRa) according to the manual provided, thereby constructing the BBD29_RS06340 deletion vector pDC24ΔBBD29_RS06340.

The sequences of the primers used in Example 1 are shown in Table 1 below.

**[Table 1]**

| Name | Sequence (5'-> 3') | SEQ ID NO |
|---|---|---|
| RS06340-5'-F | GTGAATTCGAGCTCGGTACCCATGGCAATTGA ACTGAACGTC | SEQ ID NO: 6 |
| RS06340-5'-R | CAGAGCGAGCCCGACagtactTTGCTGCACTAA TAAGGCCCC | SEQ ID NO: 7 |
| RS06340-3'-F | GCCTTATTAGTGCAGCAAagtactGTCGGGCTC GCTCTGGTG | SEQ ID NO: 8 |
| RS06340-3'-R | GGTCGACTCTAGAGGATCCCCATGTCAAACTT TGAAACGTTC | SEQ ID NO: 9 |
| RS06340-F | CTCCGAATGGGTAAACCG | SEQ ID NO: 10 |
| RS06340-R | CCTAAATTCCTCACAAGC | SEQ ID NO: 11 |

### Comparative Example 1. Construction of an L-arginine-producing microorganism in which aerobic repressor of nitrate reductase R activity is not weakened

As a control for confirming the effect of increasing L-arginine productivity by weakening aerobic repressor of nitrate reductase R activity, *Corynebacterium glutamicum* CJR2 strain, in which the argR gene was deleted and an argB (M54V) gene mutation was introduced into the *Corynebacterium glutamicum* ATCC13869 strain, and *Corynebacterium glutamicum* CJR100 strain, in which the argR gene was deleted and the argB(M54V) gene mutation and enhancement of the argC gene were introduced, were constructed as follows (Ikeda, Masato et al., Applied and environmental microbiology 75(6)1635-41, 2009).

### Comparative Example 1-1. Construction of a microorganism in which the argR gene is deleted and the argB(M54V) mutation is introduced

Vectors for argR deletion and argB(M54V) mutation introduction were constructed as follows. PCR using primer pairs of SEQ ID NOs: 16 and 17 and SEQ ID NOs: 18 and 19 and overlapping PCR using the primer pair of SEQ ID NOs: 16 and 19 were performed using the genomic DNA of *Corynebacterium glutamicum* ATCC13869 as a template to obtain a homologous recombination fragment having an argR deletion mutation sequence. To prepare a homologous recombination fragment having the argB(M54V) mutation in the same manner as above, PCR using primer pairs of SEQ ID NOs: 20 and 21 and SEQ ID NOs: 22 and 23 and overlapping PCR using the primer pair of SEQ ID NOs: 20 and 23 were performed. The PCR reaction was performed by repeating 30 times a process of denaturation at 95°C for 30 seconds; annealing at 55°C for 30 seconds; and extension at 72°C for 2 minutes. Then, the linearized pDC24 vector and each homologous recombination fragment were fusion cloned in the same manner as the method described in Example 1. The constructed vectors (recombinant plasmids) were named pDC24-ΔargR and pDC24-argB(M54V), respectively.

Then, in order to introduce an argR deletion mutation into wild-type *Corynebacterium glutamicum* ATCC13869, the *Corynebacterium glutamicum* ATCC13869 strain was transformed via homologous recombination on the chromosome using the prepared pDC24-ΔargR plasmid, thereby obtaining a recombinant strain (van der Rest et al., Appl Microbiol Biotechnol 52:541-545, 1999). PCR was performed on the obtained recombinant strain using the primer pair of SEQ ID NOs: 16 and 19 to confirm that a deletion mutation was introduced into the argR gene on the chromosome. The PCR reaction was performed by repeating 30 cycles of a process comprising: denaturation at 95°C for 30 seconds; annealing at 55°C for 30 seconds; and extension at 72°C for 2 minutes. The transformant was named CJR1.

The argB(M54V) mutation was introduced into the *Corynebacterium glutamicum* CJR1 in the same manner as above. The prepared pDC24-argB(M54V) plasmid was used, and PCR was performed using a primer pair (SEQ ID NOs: 20 and 23) for the transformant for which the secondary recombination was completed, thereby confirming that the M54V mutation was introduced into the argB gene on the chromosome, and the transformant was named *Corynebacterium glutamicum* CJR2.

The sequences of the primers used in Comparative Example 1 are shown in Table 2 below.

**[Table 2]**

| Name | Sequence (5'-> 3') | SEQ ID NO |
|---|---|---|
| *argR*-5'-F | tgaattcgagctcggtaccccactggtgaactccttgtcc | SEQ ID NO: 16 |
| *argR*-5'-R | ttgaactaggggcgctttaaaagttttccggtgttgacgg | SEQ ID NO: 17 |
| *argR*-3'-F | ccgtcaacaccggaaaacttttaaagcgcccctagttcaa | SEQ ID NO: 18 |
| *argR*-3'-R | qtcqactctaqaqgatcccccgttgaactgcttgccaqcc | SEQ ID NO: 19 |
| *argB*-5'-F | tgaattcgagctcggtaccctgcgqctcgcacggttgctc | SEQ ID NO: 20 |
| *argB*-5'-R | acggtgcgcaagaagaccacgtcggcagcaaaagcagcct | SEQ ID NO: 21 |
| *argB*-3'-F | ggctgcttttgctgccgacgtggtcttcttgcgcaccgtg | SEQ ID NO: 22 |
| *argB*-3'-R | gtcgactctagaggatccccctcttatcaggccaatcggt | SEQ ID NO: 23 |

### Comparative Example 1-2. Construction of a microorganism in which the argR gene is deleted and the argB(M54V) gene mutation and enhancement of the argC gene are introduced

Using the *Corynebacterium glutamicum* CJR2 strain constructed in Comparative Example 1-1, a CJR100 strain with improved L-arginine productivity was constructed by further enhancing the N-acetyl-gamma-glutamyl-phosphate reductase gene (hereinafter, argC).

Specifically, in order to enhance the activity of argC (SEQ ID NO: 24, NCBI accession number BBD29_RS07535), which is an N-acetyl-gamma-glutamyl-phosphate reductase gene, a plasmid for enhancing argC activity was constructed by replacing the wild-type promoter of the argC gene with Po2, using the o2 promoter (Korean Patent No. 10-1632642, hereinafter Po2) known as a strong promoter. The upstream and downstream regions of the argC gene were obtained. More specifically, to construct a strain into which argC with Po2 is introduced, PCR was performed using the chromosomal DNA of *Corynebacterium glutamicum* ATCC13869 as a template to amplify a gene fragment of the upstream region of the argC gene using the primers of SEQ ID NO: 27 and SEQ ID NO: 28 and a gene fragment of the downstream region of the argC gene using the primers of SEQ ID NO: 29 and SEQ ID NO: 30, respectively. In addition, an o2 promoter fragment was obtained using a primer pair of SEQ ID NO: 31 and SEQ ID NO: 32 with pDC24-Po2 as the template.

PfuUltra^{™} high-fidelity DNA polymerase (Stratagene) was used as a polymerase for the PCR reaction, and PCR was performed under the following conditions: denaturation at 95°C for 30 seconds; annealing at 55°C for 30 seconds; and a polymerization reaction at 72°C for 1 minute, and the denaturation, annealing, and polymerization reactions under the above conditions were repeated 28 times. As a result, an 86 bp DNA fragment of the o2 promoter region, a 610 bp DNA fragment of the upstream region of *Corynebacterium glutamicum* ATCC13869 argC, and a 1086 bp DNA fragment of the downstream region were respectively obtained. PCR was performed with primers of SEQ ID NO: 24 and SEQ ID NO: 25 using the amplified promoter fragment and DNA fragments as templates. As PCR conditions, after denaturation at 95°C for 5 minutes, denaturation at 95°C for 30 seconds; annealing at 55°C for 30 seconds; and polymerization at 72°C for 2 minutes were repeated 28 times, and then a polymerization reaction was performed at 72°C for 5 minutes. The PCR fragment obtained above was subjected to DNA purification and then ligated to a pDC24 plasmid treated with Smal restriction enzyme using an In-Fusion^{®} HD cloning kit (Clontech) for fusion cloning. The resulting vector was named pDC24-Po2-argC.

Then, the CJR2 strain produced in Comparative Example 1 was transformed using the prepared pDC24-Po2_argC plasmid by homologous recombination on the chromosome to obtain a recombinant strain (van der Rest et al., Appl Microbiol Biotechnol 52:541-545, 1999). PCR was performed on the obtained recombinant strain using the primer pair of SEQ ID NOs: 31 and 30 to confirm that the argC gene on the chromosome was enhanced by Po2. At this time, the PCR reaction was performed by repeating 30 times a process of denaturation at 95°C for 30 seconds, annealing at 55°C for 30 seconds, and extension at 72°C for 2 minutes. The transformant was named *Corynebacterium glutamicum* CJR100.

The sequences of the primers used in Comparative Example 1-2 are shown in Table 3 below.

**[Table 3]**

| Name | Sequence (5'-> 3') | SEQ ID NO |
|---|---|---|
| *argC*-5'-F | - GTGAATTCGAGCTCGGTACCCGCCCCGAAAA GCCGTTAAAAG | SEQ ID NO: 27 |
| *argC*-5'-R | tgccaaaattcacgattattgCCCACCTACAGCTAAAAC TGC | SEQ ID NO: 28 |
| *argC*-3'-F | ttattggaggagatcaaaacaATGACAATCAAGGTTGC AATC | SEQ ID NO: 29 |
| *argC*-3'-R | TTAAGGTGCGACGCCGACCTGGGGGATCCTC TAGAGTCGACC | SEQ ID NO: 30 |
| Po2-F | caataatcgtgaattttggca | SEQ ID NO: 31 |
| Po2-R | tgttttgatctcctccaataa | SEQ ID NO: 32 |

### Example 2. Construction of an L-arginine-producing microorganism having weakened aerobic repressor of nitrate reductase R activity, and evaluation of L-arginine production ability

### Example 2-1. Construction of an L-arginine-producing microorganism in which aerobic repressor of nitrate reductase R activity is weakened

In order to construct an L-arginine-producing microorganism in which aerobic repressor of nitrate reductase R activity is weakened, the *Corynebacterium glutamicum* CJR100 strain constructed in Comparative Example 1-2 was transformed using the pDC24ΔBBD29_RS06340 plasmid prepared in Example 1 by homologous recombination on the chromosome, thereby obtaining a recombinant strain (van der Rest et al., Appl Microbiol Biotechnol 52:541-545, 1999). PCR was performed on the obtained recombinant strain using the primer pair of SEQ ID NOs: 10 and 11 to confirm that the aerobic repressor of nitrate reductase R (*arnR*) was deleted. At this time, the PCR reaction was performed by repeating 30 cycles of a process of denaturation at 95°C for 30 seconds, annealing at 55°C for 30 seconds, and extension at 72°C for 2 minutes. The transformant was named *Corynebacterium glutamicum* CJR1005 (CJR100ΔarnR).

### Example 2-2. Evaluation of L-amino acid production ability of an L-arginine-producing microorganism in which aerobic repressor of nitrate reductase R activity is weakened

In order to confirm the effect of the weakening of aerobic repressor of nitrate reductase R activity on L-arginine production ability, the *Corynebacterium glutamicum* CJR1005 strain having L-arginine production ability in which aerobic repressor of nitrate reductase R activity is weakened, which was constructed in Example 2-1, and the *Corynebacterium glutamicum* CJR2 strain and *Corynebacterium glutamicum* CJ100 strain in which aerobic repressor of nitrate reductase R activity is not weakened, which were constructed in Comparative Example 1-1 and Comparative Example 1-2, were cultured by the following method to confirm the L-arginine production ability.

Specifically, the *Corynebacterium glutamicum* CJR2, *Corynebacterium glutamicum* CJ100, and *Corynebacterium glutamicum* CJR1005 strains were respectively inoculated into 250 ml corner-baffle flasks containing 25 ml of the following production medium, and shaking culture was performed at 30°C for 44 hours at 200 rpm. The composition of the production medium is as follows.

### <Production Medium (pH 7.2)>

50 g of sucrose, 57 g of ammonium sulfate, 2 g of magnesium sulfate heptahydrate, 5 g of beet molasses, 1 mg of calcium chloride, 1 mg of cobalt chloride, 2 g of monopotassium phosphate, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate, and 30 g of calcium carbonate (based on 1 liter of distilled water)

After the completion of the culture, the production ability (concentration) of L-arginine was measured using HPLC (Waters 2478). The above experiment was repeated three times, and the average L-arginine concentration value of the analysis results is shown in Table 4 below.

**[Table 4]**

| Strain name | L-arginine (g/ℓ) |
|---|---|
| CJR2 | 5.12 |
| CJR100 | 5.87 |
| CJR1005(CJR100Δ*arnR*) | 5.91 |

As a result, as shown in Table 4, it was confirmed that the L-arginine-producing *Corynebacterium glutamicum* CJR1005 strain, in which aerobic repressor of nitrate reductase R activity is weakened, exhibits L-arginine production ability enhanced by approximately 1% compared to the *Corynebacterium glutamicum* CJR100 strain, in which aerobic repressor of nitrate reductase R activity is not weakened.

From the above results, it was confirmed that a mutation introducing only the weakening of aerobic repressor of nitrate reductase R activity does not significantly affect L-arginine productivity in a microorganism of the genus *Corynebacterium.*

### Example 3. Construction of an L-arginine-producing microorganism in which glutamine synthetase activity is enhanced and evaluation of L-arginine production ability

### Example 3-1. Construction of a plasmid for introduction of a glutamine synthetase gene

In order to confirm the effect of enhancement of the activity of glutamine synthetase (type I glutamate-ammonia ligase) on L-arginine production, a recombinant plasmid vector into which a glutamine synthetase (BBD29_RS10525) gene (hereinafter, *glnA*) derived from *Corynebacterium glutamicum* ATCC13869 was introduced was constructed as follows.

Specifically, to enhance the glutamine synthetase gene *(glnA)* in the *Corynebacterium glutamicum* chromosome, BBD29_RS15405 (SEQ ID NO: 37), known as a gene encoding a transposon in *Corynebacterium glutamicum,* was used as an insertion site (Journal of Biotechnology 104, 5-25, Jorn Kalinowski et al., 2003). In order to replace the BBD29_RS10525 gene with an enhanced form, a vector for BBD29_RS15405 deletion and target gene insertion was constructed. To construct the vector, PCR was performed using the chromosome of *Corynebacterium glutamicum* ATCC13869 as a template and primer pairs of SEQ ID NOs: 39 and 40, and SEQ ID NOs: 41 and 42.

PfuUltra^{™} high-fidelity DNA polymerase (Stratagene) was used as the polymerase for the PCR reaction. The PCR conditions were as follows: denaturation at 95°C for 30 seconds, annealing at 55°C for 30 seconds, and polymerization at 72°C for 1 minute. The denaturation, annealing, and polymerization reactions were repeated 28 times to obtain DNA fragments of 791 bp and 804 bp, respectively. The obtained DNA products were purified using a PCR purification kit (QIAGEN), and the purified DNA fragments were treated with the restriction enzyme Smal and then cloned using a pDC24 vector (SEQ ID NO: 80), which had been heat-treated at 65°C for 20 minutes, and an In-Fusion Cloning Kit (TaKaRa) according to the provided manual, thereby constructing the BBD29_RS15405 deletion vector pDC24ΔBBD29_RS15405.

Then, in order to further enhance the activity of glutamine synthetase (GInA), a plasmid for further enhancing the *glnA* gene was constructed using the lysCP1 promoter (Korean Patent No. 10-0930203) (SEQ ID NO: 45), which is a promoter known as a strong promoter.

Specifically, PCR was performed to obtain a *glnA* gene fragment using the primer pair of SEQ ID NOs: 48 and 49, using the chromosome of *Corynebacterium glutamicum* ATCC13869 as a template.

PfuUltra^{™} high-fidelity DNA polymerase (Stratagene) was used as a polymerase for the PCR reaction. The PCR conditions were as follows: denaturation at 95°C for 30 seconds; annealing at 55°C for 30 seconds; and polymerization at 72°C for 1 minute. The denaturation, annealing, and polymerization were repeated 28 times to obtain a 386 bp DNA fragment of the lysCP1 promoter region and a 1434 bp DNA fragment of the *glnA* gene derived from *Corynebacterium glutamicum* ATCC13869, respectively.

PCR was performed using the primers of SEQ ID NO: 46 and 49 with the promoter region DNA fragment and the *glnA* gene DNA fragment as templates. As PCR conditions, after denaturation at 95°C for 5 minutes, denaturation at 95°C for 30 seconds; annealing at 55°C for 30 seconds; and polymerization at 72°C for 2 minutes were repeated 28 times, and then a polymerization reaction was performed at 72°C for 5 minutes to obtain an 1820 bp lysCP1_glnA(C.gl) DNA fragment containing the lysCP1 promoter and the *glnA* gene derived from *Corynebacterium glutamicum* ATCC13869.

The obtained DNA fragment was purified using a PCR purification kit (QIAGEN) and used as an insert DNA fragment for vector construction. The purified DNA fragments were treated with the restriction enzyme Scal, and then mixed with the pDC24Δ BBD29_RS15405 vector, which had been heat-treated at 65°C for 20 minutes, such that the molar ratio (M) of the vector to the insert DNA fragment was 1:2. Cloning was performed using an In-Fusion^{®} HD cloning kit (Clontech) according to the provided manual, thereby obtaining a plasmid expressing the *glnA* gene. The vector containing the *glnA* gene derived from *Corynebacterium glutamicum* ATCC13869 and the lysCP1 promoter was named "pDC24-ΔBBD29_RS15405::IysCP1_glnA(C.gl)".

The sequences of the primers used in Example 3-1 are shown in Table 5 below.

**[Table 5]**

| Name | Sequence (5'-> 3') | SEQ ID NO |
|---|---|---|
| RS15405-5'-F | aacgacggccagtgaattcGCTCGAATGCCTGACTGA CA | SEQ ID NO:39 |
| RS15405-5'-R | gtttAGTACTaaaccggaagggccAAAGGACGACTTC ACGGTTA | SEQ ID NO:40 |
| RS15405-3'-F | ccggtttAGTACTaaacaggaagagccATTGAGGATGC GAAACTGT | SEQ ID NO:41 |
| RS15405-3'-R | tgcatgcctgcaggtcgacGTAATCGAATCACCGGCCA G | SEQ ID NO:42 |
| RS15405-F | ACGTGTGCTGACTTCTCATG | SEQ ID NO:43 |
| RS15405-R | CTCATTAGTGCAAAGGTATC | SEQ ID NO:44 |
| lysCP1-F | GTCGTCCTTTGGCCCTTCCGGTTTAGTggcccttc cggtttagt | SEQ ID NO: 46 |
| lysCP1-R | TTCCGGGGTTTCAAACGCCATatgtgtgcacctttcgat ctacg | SEQ ID NO: 47 |
| *glnA*(C.gl) -F | tagatcgaaaggtgcacacatATGGCGTTTGAAACCCC GGAA | SEQ ID NO: 48 |
| *glnA*(C.gl)-R | CCTCAATGGCTCTTCCTGTTTAGTTTAGCAGTC GAAGTACAATTC | SEQ ID NO: 49 |

### 3-2. Construction of an L-arginine-producing microorganism in which glutamine synthetase activity is enhanced

In order to confirm the effect of enhancement of glutamine synthetase activity on L-arginine productivity in a *Corynebacterium glutamicum* strain having L-arginine production ability, a strain in which the activity of glutamine synthetase (GInA) is enhanced by further introducing the *glnA* gene was constructed by the following method.

Specifically, the pDC24-ΔBBD29_RS15405::IysCP1_glnA(C.gl) vector prepared in Example 3-1 was transformed into the *Corynebacterium glutamicum* CJR100 strain, which is an L-arginine-producing strain constructed in Comparative Example 1-2. A recombinant strain was obtained by transforming the *Corynebacterium glutamicum* CJR100 strain via homologous recombination on the chromosome (van der Rest et al., Appl Microbiol Biotechnol 52:541-545, 1999). PCR was performed on the obtained recombinant strain using the primer pair of SEQ ID NOs: 43 and 43 to confirm that the glutamine synthetase gene had been introduced. At this time, the PCR reaction was performed by repeating 30 cycles of a process of denaturation at 95°C for 30 seconds, annealing at 55°C for 30 seconds, and extension at 72°C for 2 minutes. The transformant was named CJR1007 (CJR100LlBBD29_RS15405::lysCP1_glnA(C.gl)).

### 3-3. Evaluation of L-arginine production ability of the L-arginine-producing microorganism in which the activity of the glutamine synthetase is enhanced

The *Corynebacterium glutamicum* CJR1007 constructed in Example 3-2 and the *Corynebacterium glutamicum* CJR100 strain, which is a parent strain, were subjected to flask culture by the following method to analyze the L-arginine production ability.

Specifically, each strain was inoculated into a 250 ml corner-baffle flask containing 25 ml of seed medium, and shaking culture was performed at 30°C for 44 hours at 200 rpm. After the completion of the culture, the production amount (concentration) of L-arginine was measured using HPLC (Waters 2478). The above experiment was repeated three times, and the average value of the analysis results is shown in Table 6 below.

### <Production Medium (pH 7.2)>

50 g of sucrose, 57 g of ammonium sulfate, 2 g of magnesium sulfate heptahydrate, 5 g of beet molasses, 1 mg of calcium chloride, 1 mg of cobalt chloride, 2 g of monopotassium phosphate, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate, and 30 g of calcium carbonate (based on 1 liter of distilled water)

**[Table 6]**

| Strain name | L-arginine (g/ℓ) |
|---|---|
| CJR100 | 5.87 |
| CJR1007(CJR100Δ*BBD29_RS15405*::lysCP1_*glnA*(C.gl) | 5.86 |

As a result, as shown in Table 6, it was confirmed that the L-arginine strain having enhanced glutamine synthetase activity produces L-arginine at a level equivalent to that of the parent strain *Corynebacterium glutamicum* CJR100, in which the glutamine synthetase activity is not enhanced.

### Example 3-4. Construction of a plasmid for introduction of a foreign glutamine synthetase gene

In order to confirm the effect of the introduction of a foreign heterologous glutamine synthetase gene on L-arginine production, a recombinant plasmid vector for introducing a glutamine synthetase gene (*glnA*) derived from Aureibacillus halotolerans and a glutamine synthetase gene (*glnA*) derived from Bacillus subtilis subsp. 168 was constructed as follows.

Specifically, in order to insert a foreign glutamine synthetase (BBD29_RS10525) gene (*glnA*) into the *Corynebacterium glutamicum* chromosome, the pDC24ΔBBD29_RS15405 vector prepared in Example 3-1 was used as an insertion site. In order to further enhance the activity of glutamine synthetase (GInA), a plasmid for further enhancing the expression of the *glnA* gene was constructed using the lysCP1 promoter (Korean Patent No. 10-0930203) (SEQ ID NO: 45), which is known as a strong promoter.

Specifically, to construct a vector containing a gene into which a foreign *glnA* gene having a lysCP1 promoter has been introduced, PCR was performed using the chromosomes of Aureibacillus halotolerans (A.ht) and Bacillus subtilis subsp. 168 (B.su) as templates, respectively; specifically, a primer pair of SEQ ID NOs: 50 and 51 was used to obtain a *glnA* gene fragment derived from Aureibacillus halotolerans (A.ht) (SEQ ID NO: 34), and a primer pair of SEQ ID NOs: 52 and 53 was used to obtain a *glnA* gene fragment derived from Bacillus subtilis subsp. 168 (B.su) (SEQ ID NO: 36).

PfuUltra^{™} high-fidelity DNA polymerase (Stratagene) was used as a polymerase for the PCR reaction, and PCR was performed under the following conditions: denaturation at 95°C for 30 seconds; annealing at 55°C for 30 seconds; and a polymerization reaction at 72°C for 1 minute, and the denaturation, annealing, and polymerization reactions were repeated 28 times to obtain a 386 bp DNA fragment of the lysCP1 promoter region, a 1338 bp DNA fragment of the *glnA* gene derived from Aureibacillus halotolerans, and a 1335 bp DNA fragment of the *glnA* gene derived from Bacillus subtilis subsp. 168, respectively.

PCR was performed using the primers of SEQ ID NOs: 46 and 51 and the primers of SEQ ID NOs: 46 and 53 with the promoter region DNA fragment and each *glnA* gene DNA fragment as templates. As PCR conditions, after denaturation at 95°C for 5 minutes, denaturation at 95°C for 30 seconds; annealing at 55°C for 30 seconds; and polymerization at 72°C for 2 minutes were repeated 28 times, and then a polymerization reaction was performed at 72°C for 5 minutes to obtain a 1724 bp lysCP1_glnA(A.ht) DNA fragment containing the lysCP1 promoter and the *glnA* gene derived from Aureibacillus halotolerans and a 1721 bp lysCP1_glnA(B.su) DNA fragment containing the lysCP1 promoter and the *glnA* gene derived from Bacillus subtilis subsp. 168.

The obtained DNA fragments were purified using a PCR purification kit (QIAGEN) and used as insert DNA fragments for vector construction. After treating the purified DNA fragments with the restriction enzyme Scal, the molar ratio of the pDC24ΔBBD29_RS15405 vector, which had been heat-treated at 65°C for 20 minutes, to the insert DNA fragments was set to 1:2. Cloning was performed using the In-Fusion^{®} HD cloning kit (Clontech) according to the provided manual, thereby obtaining plasmids, each expressing a *glnA* gene. The vector containing the *glnA* gene derived from Aureibacillus halotolerans and the lysCP1 promoter was named "pDC24-ΔBBD29_RS15405::IysCP1_glnA(A.ht)", and the vector containing the glnA gene derived from Bacillus subtilis subsp. 168 and the lysCP1 promoter was named "pDC24-ΔBBD29_RS15405::IysCP1_glnA(B.su)".

The sequences of the primers used in Example 3-4 are described in Table 7 below.

**[Table 7]**

| Name | Sequence (5'-> 3') | SEQ ID NO |
|---|---|---|
| *glnA*(A.ht)-F | tagatcgaaaggtgcacacatATGGGTTCTAAGTACACGAG AAAAG | SEQ ID NO: 50 |
| *glnA*(A.ht)-R | CTCTTCCTGTTTAGTTTAGTAAAGGGTAAGGTACTG C | SEQ ID NO: 51 |
| *glnA*(B.su)-F | tagatcgaaaggtgcacacatATGGCAAAGTACACTAGAGA AGATA | SEQ ID NO: 52 |
| *glnA*(B.su)-R | CTCTTCCTGTTTAGTTTAATACTGAGACATATACTGT TCG | SEQ ID NO: 53 |

### Example 3-5. Construction of an L-arginine-producing microorganism in which foreign glutamine synthetase activity is enhanced

In order to confirm the effect of enhancement of glutamine synthetase activity according to the introduction of a foreign heterologous glutamine synthetase gene on L-arginine production in a *Corynebacterium glutamicum* strain having L-arginine production ability, a strain into which a foreign-derived *glnA* gene was introduced was constructed by the following method.

Specifically, the *Corynebacterium glutamicum* CJR100 strain prepared in Comparative Example 1-2 was transformed by homologous recombination on the chromosome using the pDC24-ΔBBD29_RS15405::IysCP1_glnA(A.ht) and pDC24-ΔBBD29_RS15405::lysCP1_glnA(B.su) plasmids prepared in Examples 3-4, respectively, to obtain recombinant strains (van der Rest et al., Appl Microbiol Biotechnol 52:541-545, 1999). PCR was performed on the obtained recombinant strains using a primer pair of SEQ ID NOs: 43 and 43 to confirm that the glutamine synthetase gene was introduced. At this time, the PCR reaction was performed by repeating 30 cycles of a process of denaturation at 95°C for 30 seconds, annealing at 55°C for 30 seconds, and extension at 72°C for 2 minutes. The transformants were named CJR1008 (CJR100ΔBBD29_RS15405::lysCP1_glnA(A.ht)) and CJR1009 (CJR100ΔBBD29_RS15405::lysCP1_glnA(B.su)).

### Example 3-6. Evaluation of L-arginine productivity of the L-arginine-producing microorganism into which the foreign glutamine synthetase gene is introduced

In order to evaluate the L-arginine productivity of an L-arginine-producing microorganism into which a foreign glutamine synthetase gene has been introduced, the L-arginine production ability of the *Corynebacterium glutamicum* CJR1008 (CJR100ΔBBD29_RS15405::lysCP1_glnA(A.ht)) and the *Corynebacterium glutamicum* CJR1009 (CJR100ΔBBD29_RS15405::lysCP1_glnA(B.su)) prepared in Example 3-5, and the *Corynebacterium glutamicum* CJ100 prepared in Comparative Example 1-2, was confirmed in the same manner as in Example 3-3, and the results are shown in Table 8 below.

**[Table 8]**

| Strain name | L-arginine (g/ℓ) |
|---|---|
| CJR100 | 5.87 |
| CJR100Δ *BBD29_RS15405* | 5.86 |
| CJR1007 (CJR100Δ*BBD29_RS15405*::lysCP1_*glnA*(C.gl) | 5.86 |
| CJR1008(CJR100Δ*BBD29_RS15405*::lysCP1_*glnA*(A.ht) | 5.90 |
| CJR1009(CJR100Δ*BBD29_RS15405*::lysCP1_*glnA*(B.su) | 5.84 |

As a result, as shown in Table 8, it was confirmed that the L-arginine production ability of the strain into which the foreign glutamine synthetase gene was introduced showed a level equivalent to that of the parent strain, *Corynebacterium glutamicum* CJR100.

### Example 4. Construction of an L-arginine-producing microorganism in which aerobic repressor of nitrate reductase R activity is weakened and glutamine synthetase activity is enhanced, and evaluation of L-arginine production ability

### Example 4-1. Construction of an L-arginine-producing microorganism in which aerobic repressor of nitrate reductase R activity is weakened and glutamine synthetase activity is simultaneously enhanced

In order to confirm the effect of a combination mutation of weakening of aerobic repressor of nitrate reductase R activity and enhancement of glutamine synthetase activity on L-arginine production ability, strains in which *arnR* was deleted and a *glnA* gene was additionally introduced were constructed by the following method.

Specifically, the *Corynebacterium glutamicum* CRJ1005 strain in which aerobic repressor of nitrate reductase R activity is weakened, which was prepared in Example 2-1, was transformed using the pDC24-ΔBBD29_RS15405::lysCP1_glnA(C.gl) vector prepared in Example 3-1, and the pDC24-ΔBBD29_RS15405::IysCP1_glnA(A.ht) vector and pDC24-ΔBBD29_RS15405::lysCP1_glnA(B.su) vector prepared in Example 3-4 by homologous recombination on the chromosome to obtain recombinant strains (van der Rest et al., Appl Microbiol Biotechnol 52:541-545, 1999). PCR was performed on the obtained recombinant strains using the primer pair of SEQ ID NOs: 43 and 44 to confirm that the glutamine synthetase gene was introduced. In this process, the PCR reaction was performed by repeating 30 cycles of denaturation at 95°C for 30 seconds, annealing at 55°C for 30 seconds, and extension at 72°C for 2 minutes. The transformants were named CJR1013 (CJR1005ΔΔBBD29_RS15405::lysCP1_glnA(B.su)), CJR1014 (CJR1005ΔΔBBD29_RS15405::lysCP1_glnA(A.ht)), and CJR1019 (CJR1005ΔΔBBD29_RS15405::lysCP1_glnA(C.gl)), respectively.

### Example 4-2. Confirmation of the effect of increasing L-arginine productivity in an L-arginine-producing microorganism in which aerobic repressor of nitrate reductase R activity is weakened and glutamine synthetase activity is enhanced

The *Corynebacterium glutamicum* CJR1013, CJR1014, and CJR1019 strains prepared in Example 4-1, the *Corynebacterium glutamicum* CJR100 strain prepared in Comparative Example 1-1, the *Corynebacterium glutamicum* CRJ1005 strain prepared in Example 2-1, the *Corynebacterium glutamicum* CJR1007 strain prepared in Example 3-2, and the *Corynebacterium glutamicum* CJR1008 and CJR1009 strains prepared in Example 3-5 were subjected to flask culture by the following method to analyze the L-arginine production ability.

Specifically, each strain was inoculated into a 250 ml corner-baffle flask containing 25 ml of seed medium, and shaking culture was performed at 30°C for 44 hours at 200 rpm. After the completion of culturing, the production amount (concentration) of L-arginine in each strain culture solution was measured using HPLC (Waters 2478). The above experiment was repeated three times, and the average value of the analysis results is shown in Table 9 below.

### <Production Medium (pH 7.2)>

50 g of sucrose, 57 g of ammonium sulfate, 2 g of magnesium sulfate heptahydrate, 5 g of beet molasses, 1 mg of calcium chloride, 1 mg of cobalt chloride, 2 g of monopotassium phosphate, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate, and 30 g of calcium carbonate (based on 1 liter of distilled water)

**[Table 9]**

| Strain name | L-arginine (g/ℓ) |
|---|---|
| CJR100 | 5.87 |
| CJR100Δ *BBD29_RS15405* | 5.86 |
| CJR1005(CJR100Δ*arnR*) | 5.91 |
| CJR1007 (CJR100Δ*BBD29_RS15405::lysCP1_glnA*(C.gl) | 5.86 |
| CJR1008(CJR100Δ*BBD29_RS15405*::lysCP1_*glnA*(A.ht*)* | 5.90 |
| CJR1009(CJR100Δ*BBD29_RS15405*::lysCP1_*glnA*(B.su) | 5.84 |
| CJR1019(CJR1005Δ*BBD29_RS15405*::lyscP1_*glnA*(C.gl) | 6.10 |
| CJR1014(CJR1005Δ*BBD29_RS15405*::lysCP1_*glnA*(A.ht) | 6.30 |
| CJR1013 (CJR1005Δ*BBD29_RS15405*::lysCP1_*glnA*(B.su) | 6.17 |

As a result, as shown in Table 9, it was confirmed that the L-arginine production abilities of the *Corynebacterium glutamicum* CJR1019, CJR1014, and CJR1013 strains, in which aerobic repressor of nitrate reductase R activity is weakened and glutamine synthetase activity is simultaneously enhanced, were improved by 4%, 7%, and 5%, respectively, compared to the parent strain CJR100. In addition, it was confirmed that the L-arginine production abilities of the *Corynebacterium glutamicum* strains, in which aerobic repressor of nitrate reductase R activity is weakened and glutamine synthetase activity is simultaneously enhanced, were increased compared to the *Corynebacterium glutamicum* CJR1005 strain, which is a strain in which only aerobic repressor of nitrate reductase R activity is weakened, and the *Corynebacterium glutamicum* CJR1007, CJR1008, and CJR1009 strains, in which only glutamine synthetase activity is enhanced.

From the above results, it was confirmed that when an *arnR* deletion single mutation and a *glnA* enhancement single mutation are introduced into a microorganism of the genus *Corynebacterium,* the L-arginine production ability is at a level equivalent to that of the parent strain; however, when the *glnA* enhancement mutation is introduced simultaneously with the *arnR* deletion, the L-arginine production ability of the microorganism of the genus *Corynebacterium* is enhanced, which is effective for L-arginine production.

### Example 5. Construction of a plasmid for inserting a gene complex encoding malate:quinone oxidoreductase, malate dehydrogenase, aspartate transaminase, NADP-specific glutamate dehydrogenase, and fumarate hydratase

In order to confirm the effect of improving L-arginine productivity by the additional introduction of a complex comprising all of the following genes of *Corynebacterium glutamicum:* a gene (SEQ ID NO: 55, hereinafter *mqo*) encoding malate:quinone oxidoreductase (SEQ ID NO: 54), a gene (SEQ ID NO: 59, hereinafter *mdh*) encoding malate dehydrogenase (SEQ ID NO: 58), a gene (SEQ ID NO: 61, hereinafter *aspB*) encoding aspartate transaminase (SEQ ID NO: 60), a gene (SEQ ID NO: 63, hereinafter *gdh*) encoding NADP-specific glutamate dehydrogenase (SEQ ID NO: 62), and a gene (SEQ ID NO: 65, hereinafter *fumC*) encoding fumarate hydratase (SEQ ID NO: 64), a complex enhancement vector was constructed in the following manner.

Specifically, to insert a gene complex into the *Corynebacterium glutamicum* chromosome, the mqo gene, which is a component of the complex, was used as an insertion site. In order to achieve replacement with the complex within the *mqo* gene, a target gene insertion vector containing the *mqo* gene was constructed. PCR was performed using the chromosome of the *Corynebacterium glutamicum* ATCC13032 strain as a template and using primer pairs for each gene of the complex described in Table 10.

PfuUltra^{™} high-fidelity DNA polymerase (Stratagene) was used as a polymerase for the PCR reaction. The PCR was performed under the following conditions: denaturation at 95°C for 30 seconds, annealing at 55°C for 30 seconds, and polymerization at 72°C for 1 minute; and these denaturation, annealing, and polymerization steps were repeated 28 times. As a result, DNA fragments were obtained as follows: an mqo gene fragment of 1947 bp using a primer pair of SEQ ID NOs: 66 and 67, an mdh gene fragment of 1357 bp using a primer pair of SEQ ID NOs: 68 and 69, an aspB gene fragment of 1575 bp using a primer pair of SEQ ID NOs: 70 and 71, a gdh gene fragment of 1794 bp using a primer pair of SEQ ID NOs: 72 and 73, a fumC gene fragment of 1686 bp using a primer pair of SEQ ID NOs: 74 and 75, and an mqo gene fragment of 1916 bp using a primer pair of SEQ ID NOs: 76 and 77. Subsequently, PCR was performed using the obtained DNA products as templates using the primers of SEQ ID NO: 66 and SEQ ID NO: 77. PfuUltra^{™} high-fidelity DNA polymerase (Stratagene) was used as the polymerase, and the PCR conditions were denaturation at 95°C for 30 seconds, annealing at 55°C for 30 seconds, and polymerization at 72°C for 10 minutes, with the denaturation, annealing, and polymerization steps being repeated 28 times. As a result, a 10275 bp mqo-mdh-aspB-gdh-fumC-mqo gene complex DNA fragment was obtained. The obtained DNA product was purified using a PCR purification kit (QUIAGEN), and the purified amplification product was treated with restriction enzyme Smal. Then, a pDC24 *mqo-mdh-aspB-gdh-fumC-mqo* vector for introducing *mqo-mdh-aspB-gdh-fumC-mqo* into the chromosome was prepared using the treated amplification product, a pDC24 vector (SEQ ID NO: 80) that had been heat-treated at 65°C for 20 minutes, and an Infusion cloning kit (TaKaRa) in accordance with the provided manual.

The sequences of the primers used in Example 5 are shown in Table 10 below.

**[Table 10]**

| Name | Sequence (5'-> 3') | SEQ ID NO |
|---|---|---|
| *mqo*-5'-F | GTGAATTCGAGCTCGGTACCCATACTACTCATGTTT GCGAAT | SEQ ID NO: 66 |
| *mqo*-3'-R | TCCTGATATCGGGCACCATTTGGCAAAGAATACGC AAAGCAC | SEQ ID NO: 67 |
| *mdh*-5'-F | TGCTTTGCGTATTCTTTGCCAAATGGTGCCCGATAT CAGGA | SEQ ID NO: 68 |
| *mdh*-3'-R | AACAAAGCAGCCATGCGTTGCAGCCGTTACTTAAA CCAAGTC | SEQ ID NO: 69 |
| *aspB*-5'-F | CGACTTGGTTTAAGTAACGGCTGCAACGCATGGCT GCTTTGTT | SEQ ID NO: 70 |
| *aspB*-3'-R | GCGAATGAGACCAGTTGACTGTTAGTTAGCGTAAT GCTCCGC | SEQ ID NO: 71 |
| *gdh*-5'-F | GCGGAGCATTACGCTAACTAACAGTCAACTGGTCT CATTCGC | SEQ ID NO: 72 |
| *gdh*-3'-R | GGTTAATATCCAATATGGAAGTTAGATGACGCCCTG TGCCAG | SEQ ID NO: 73 |
| *fumC*-5'-F | CTGGCACAGGGCGTCATCTAACTTCCATATTGGAT ATTAACC | SEQ ID NO: 74 |
| *fumC*-3'-R | ATTCGCAAACATGAGTAGTATTTAGAACTTGTTCTC GCGCTC | SEQ ID NO: 75 |
| *mqo*-5'-F | CGCGAGAACAAGTTCTAAATACTACTCATGTTTGCG AATTG | SEQ ID NO: 76 |
| *mqo*-3'-R | GGTCGACTCTAGAGGATCCCCTTAGGCTTCCTCAA GCTTCAG | SEQ ID NO: 77 |
| Mqo-F | CTCTTCACCAGCATT | SEQ ID NO: 78 |
| Mqo-R | TTGTCACAACATCTGTTTCA | SEQ ID NO: 79 |

### Example 6. Construction of a microorganism in which activities of all of malate:quinone oxidoreductase, malate dehydrogenase, aspartate transaminase, NADP-specific glutamate dehydrogenase, and fumarate hydratase are enhanced, and evaluation of L-amino acid production ability

### Example 6-1. Construction of a microorganism in which activities of all of malate:quinone oxidoreductase, malate dehydrogenase, aspartate transaminase, NADP-specific glutamate dehydrogenase, and fumarate hydratase are enhanced

In order to construct a microorganism in which the activities of malate:quinone oxidoreductase, malate dehydrogenase, aspartate transaminase, NADP-specific glutamate dehydrogenase, and fumarate hydratase are all enhanced, recombinant strains were obtained by transforming the *Corynebacterium glutamicum* CJR100 prepared in Comparative Example 1-2 and the *Corynebacterium glutamicum* CJR1005 prepared in Example 2-1 with the pDC24 *mqo-mdh-aspB-gdh-fumC-mqo* plasmid prepared in Example 5 via homologous recombination on the chromosome (van der Rest et al., Appl Microbiol Biotechnol 52:541-545, 1999). PCR was performed on the obtained recombinant strains using the primer pair of SEQ ID NOs: 78 and 79 to confirm that the *mqo-mdh-aspB-gdh-fumC-mqo* complex was introduced into the *Corynebacterium glutamicum* chromosome. The PCR reaction was performed by repeating 30 cycles of a process of denaturation at 95°C for 30 seconds; annealing at 55°C for 30 seconds; and extension at 72°C for 2 minutes. The transformant derived from the *Corynebacterium glutamicum* CJR100 was designated as *Corynebacterium glutamicum* CJR1012, and the transformant derived from the *Corynebacterium glutamicum* CJR1005 was designated as *Corynebacterium glutamicum* CJR1015.

### Example 6-2. Evaluation of L-arginine production ability of the microorganism in which the activities of all of malate:quinone oxidoreductase, malate dehydrogenase, aspartate transaminase, NADP-specific glutamate dehydrogenase, and fumarate hydratase are enhanced

In order to confirm the effect of enhancement of the activities of malate:quinone oxidoreductase, malate dehydrogenase, aspartate transaminase, NADP-specific glutamate dehydrogenase, and fumarate hydratase on L-amino acid production ability, the *Corynebacterium glutamicum* CJR1012 and CJR1015 prepared in Example 6-1, the *Corynebacterium glutamicum* CJ100 prepared in Comparative Example 1-2, and the *Corynebacterium glutamicum* CJR1005 prepared in Example 2-1 were cultured by the following method to confirm the L-arginine production ability.

Specifically, the *Corynebacterium glutamicum* CJ100, *Corynebacterium glutamicum* CJR1005, *Corynebacterium glutamicum* CJR1012, and CJR1015 were respectively inoculated into 250 ml corner-baffle flasks containing 25 ml of the following production medium, and shaking culture was performed at 30°C for 44 hours at 200 rpm. The composition of the production medium is as follows.

### <Production Medium (pH 7.2)>

50 g of sucrose, 57 g of ammonium sulfate, 2 g of magnesium sulfate heptahydrate, 5 g of beet molasses, 1 mg of calcium chloride, 1 mg of cobalt chloride, 2 g of monopotassium phosphate, 0.01 mg of biotin, 0.1 mg of thiamine-HCl, 2 mg of calcium pantothenate, 3 mg of nicotinamide, 10 mg of ferrous sulfate, 10 mg of manganese sulfate, 0.02 mg of zinc sulfate, 0.5 mg of copper sulfate, 30 g of calcium carbonate (based on 1 liter of distilled water)

After the completion of the culture, the production ability (concentration) of L-arginine was measured using HPLC (Waters 2478). The above experiment was repeated three times, and the average value of the L-arginine concentration, which is the analysis result, is shown in Table 11 below.

**[Table 11]**

| Strain name | L-arginine (g/ℓ) |
|---|---|
| CJR100 | 5.87 |
| CJR1005(CJR100△*arnR*) | 5.91 |
| CJR1012 (CJR100- *mqo-mdh-aspB-gdh-fumC-mqo*) | 6.40 |
| CJR1015 (CJR1005- *mqo-mdh-aspB-gdh-fumC-mqo*) | 6.70 |

As a result, as shown in Table 11, it was confirmed that the L-arginine production ability of *Corynebacterium glutamicum* CJR1012 into which the *mqo-mdh-aspB-gdh-fumC-mqo* complex was introduced was improved by 9% compared to the parent strain *Corynebacterium glutamicum* CJR100, and it was confirmed that the L-arginine production ability of the *Corynebacterium glutamicum* CJR1015 in which *arnR* was deleted and into which the *mqo-mdh-aspB-gdh-fumC-mqo* complex was introduced was improved by 13% compared to the parent strain *Corynebacterium glutamicum* CJR1005.

From the above results, it was confirmed that the enhancement of the activities of malate:quinone oxidoreductase, malate dehydrogenase, aspartate transaminase, NADP-specific glutamate dehydrogenase, and fumarate hydratase increases the L-arginine production ability of a microorganism of the genus *Corynebacterium.*

### Example 6-3. Construction of an L-arginine-producing microorganism in which aerobic repressor of nitrate reductase R activity is weakened, and activities of a glutamine synthetase, malate:quinone oxidoreductase, malate dehydrogenase, aspartate transaminase, NADP-specific glutamate dehydrogenase, and fumarate hydratase are simultaneously enhanced

In order to construct an L-arginine-producing microorganism in which aerobic repressor of nitrate reductase R activity is weakened, glutamine synthetase activity is enhanced, and the activities of malate:quinone oxidoreductase, malate dehydrogenase, aspartate transaminase, NADP-specific glutamate dehydrogenase, and fumarate hydratase are all enhanced, recombinant strains were obtained by transforming the *Corynebacterium glutamicum* CJR1015 strain prepared in Example 6-1 with the pDC24-ΔBBD29_RS15405::lysCP1_glnA(C.gl) plasmid prepared in Example 3-1, the pDC24-ΔBBD29_RS15405::lysCP1_glnA(A.ht) plasmid prepared in Example 3-4, and the pDC24-ΔBBD29_RS15405::lysCP1_glnA(B.su) plasmid via homologous recombination on the chromosome (van der Rest et al., Appl Microbiol Biotechnol 52:541-545, 1999). PCR was performed on the obtained recombinant strains using the primer pair of SEQ ID NOs: 43 and 44 to confirm that the glutamine synthetase gene had been introduced into the chromosome. The PCR reaction was performed by repeating 30 cycles of a process of denaturation at 95°C for 30 seconds, annealing at 55°C for 30 seconds, and extension at 72°C for 2 minutes. The transformants were named *Corynebacterium glutamicum* CJR1016 (CJR1015ΔBBD29_RS15405::lysCP1_glnA(C.gl)), CJR1017 (CJR1015ΔBBD29_RS15405::lysCP1_glnA(B.su)), and CJR1018 (CJR1015ΔBBD29_RS15405::IysCP1_glnA(A.ht)) strains.

### Example 6-4. Confirmation of an increase in L-arginine production ability of an L-arginine-producing microorganism in which aerobic repressor of nitrate reductase R activity is weakened, and the activities of glutamine synthetase, malate:quinone oxidoreductase, malate dehydrogenase, aspartate transaminase, NADP-specific glutamate dehydrogenase, and fumarate hydratase are simultaneously enhanced

In order to confirm the effect of increasing the L-arginine production ability of an L-arginine-producing microorganism resulting from the weakening of aerobic repressor of nitrate reductase R activity, the enhancement of glutamine synthetase activity, and the enhancement of all activities of malate:quinone oxidoreductase, malate dehydrogenase, aspartate transaminase, NADP-specific glutamate dehydrogenase, and fumarate hydratase, the strains were cultured by the following method to analyze the concentration of L-arginine in the culture solution.

Specifically, the *Corynebacterium glutamicum* CJR100 strain, which is a control group, and the *Corynebacterium glutamicum* CJR1016 strain, CJR1017 strain, and CJR1018 strain prepared in Example 6-3 were inoculated into 250 ml corner-baffle flasks containing 25 ml of the production medium described in Example 6-2, and shaking culture was performed at 30°C for 44 hours at 200 rpm.

After the completion of the culture, the production ability (concentration) of L-arginine was measured using HPLC (Waters 2478). The above experiment was repeated three times, and the average L-arginine concentration value of the analysis result is shown in Table 12 below.

**[Table 12]**

| Strain name | Histidine (g/L) |
|---|---|
| CJR100 | 5.87 |
| CJR1005(CJR100Δ*arnR*) | 5.91 |
| CJR1012 (CJR100- *mqo-mdh-aspB-gdh-fumC-mqo)* | 6.40 |
| CJR1015 (CJR1005- *mqo-mdh-aspB-gdh-fumC-mqo)* | 6.70 |
| CJR1016(CJR1015△*BBD29_RS15405*::lysCP1_*gln*A(C.gl) | 6.83 |
| CJR1017(CJR1015△*BBD29_RS15405:*lysCP1_*glnA*(B.su) | 7.07 |
| CJR1018 (CJR1015△*BBD29*_*RS15405*::lysCP1_*glnA*(A.ht) | 7.43 |

As a result, as shown in Table 12, it was confirmed that the L-arginine production abilities of the CJR1016, CJR1017, and CJR1018 strains, which are L-arginine-producing strains in which aerobic repressor of nitrate reductase R activity is weakened and the activities of glutamine synthetase, malate:quinone oxidoreductase, malate dehydrogenase, aspartate transaminase, NADP-specific glutamate dehydrogenase, and fumarate hydratase are simultaneously enhanced, were improved by 16%, 20%, and 26%, respectively, compared to the *Corynebacterium glutamicum* CJR100 strain. In addition, it was confirmed that the L-arginine production abilities of these strains were improved by 2%, 5%, and 11%, respectively, compared to the *Corynebacterium glutamicum* CJR1015 strain into which the *mqo-mdh-aspB-gdh-fumC-mqo* complex was not introduced.

From the above results, it was confirmed that the weakening of aerobic repressor of nitrate reductase R activity, the enhancement of glutamine synthetase activity, and the simultaneous enhancement of the activities of malate:quinone oxidoreductase, malate dehydrogenase, aspartate transaminase, NADP-specific glutamate dehydrogenase, and fumarate hydratase increase the L-arginine production ability of a microorganism of the genus *Corynebacterium.*

From the above description, those skilled in the art to which the present disclosure pertains will understand that the present disclosure may be implemented in other specific forms without changing its technical spirit or essential features. In this regard, it should be understood that the examples described above are illustrative in all respects and not limiting. The scope of the present disclosure should be interpreted such that all changes or modifications derived from the meaning and scope of the following patent claims and their equivalent concepts are included in the scope of the present disclosure, rather than being limited to the above detailed description.

## Claims

1. A microorganism of the genus *Corynebacterium* in which aerobic repressor of nitrate reductase R activity is weakened and glutamine synthetase activity is enhanced.

2. The microorganism of the genus *Corynebacterium* according to claim 1, wherein the microorganism has L-arginine production ability.

3. The microorganism of the genus *Corynebacterium* according to claim 1, wherein the aerobic repressor of nitrate reductase R comprises the amino acid sequence of SEQ ID NO: 1.

4. The microorganism of the genus *Corynebacterium* according to claim 1, wherein the glutamine synthetase comprises the amino acid sequence of SEQ ID NO: 3, the amino acid sequence of SEQ ID NO: 33, the amino acid sequence of SEQ ID NO: 35, or an amino acid sequence having 80% or more sequence identity to any one of the amino acid sequences selected from the group consisting of the amino acid sequence of SEQ ID NO: 3, the amino acid sequence of SEQ ID NO: 33, and the amino acid sequence of SEQ ID NO: 35.

5. The microorganism of the genus *Corynebacterium* according to claim 1, wherein the aerobic repressor of nitrate reductase R activity is weakened by one or more methods selected from the group consisting of: deletion of all or part of a gene encoding a polypeptide; and substitution, modification, and gene mutation of an expression regulatory region.

6. The microorganism of the genus *Corynebacterium* according to claim 1, wherein the glutamine synthetase activity is enhanced by one or more methods selected from the group consisting of an increase in copy number by chromosomal insertion and vector introduction, and substitution, modification, and gene mutation of an expression regulatory region.

7. The microorganism of the genus *Corynebacterium* according to claim 1, wherein the microorganism of the genus *Corynebacterium* is one in which malate:quinone oxidoreductase activity, malate dehydrogenase activity, aspartate transaminase activity, and NADP-specific glutamate dehydrogenase activity are further enhanced.

8. The microorganism of the genus *Corynebacterium* according to claim 1, wherein the microorganism of the genus *Corynebacterium* is *Corynebacterium glutamicum.*

9. The microorganism of the genus *Corynebacterium* according to any one of claims 1 to 8, wherein the microorganism has an increased L-arginine production ability compared to an unmodified microorganism of the same species in which aerobic repressor of nitrate reductase R activity is not weakened or glutamine synthetase activity is not enhanced.

10. A method for producing L-arginine, comprising culturing the microorganism of the genus *Corynebacterium* according to any one of claims 1 to 8 in a medium; and recovering L-amino acids from the cultured microorganism, the medium, or both.

11. The method for producing L-arginine according to claim 10, wherein the microorganism of the genus *Corynebacterium* is *Corynebacterium glutamicum.*

12. A use of the microorganism of the genus *Corynebacterium* according to any one of claims 1 to 8 for producing L-arginine.
